# FASCICULE DE BREVET EUROPEEN

(11) **EP 2 854 915 B1**
(45) Date de publication et mention de la délivrance du brevet: **20.04.2016**
(21) Numéro de dépôt: 13741802.6
(22) Date de dépôt: 30.05.2013
(51) Int. Cl.: A61M 15/00

(54) **DISPOSITIF DE DISTRIBUTION DE PRODUIT FLUIDE.**
VORRICHTUNG ZUR AUSGABE EINES FLÜSSIGPRODUKTS
FLUID DISPENSING DEVICE

(30) Priorité: 31.05.2012 FR 1255011
(43) Date de publication de la demande: 08.04.2015
(73) Titulaire: Aptar France SAS, 27110 Le Neubourg (FR)
(72) Inventeur: BAILLET, Matthieu, 76240 Bonsecours (FR); KIRNIAK, Maxime, 76000 Rouen (FR); LAUT, Antoine, 95420 Wy Dit Joli Village (FR)
(74) Mandataire: CAPRI
(86) Numéro de dépôt international: PCT/FR2013/051217
(87) Numéro de publication internationale: WO 2013/178952

(56) Documents cités:
- WO-A1-02/085281
- FR-A1- 2 936 425
- US-B1- 6 179 164

## Description

La présente invention concerne un dispositif de distribution de produit fluide, et plus particulièrement un inhalateur de poudre sèche.

Les inhalateurs sont bien connus dans l'état de la technique. Il en existe différentes sortes. Un premier type d'inhalateur contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Des inhalateurs comportant des réservoirs individuels, tels que des capsules, qui sont à charger dans l'inhalateur juste avant l'utilisation de celui-ci ont également été décrits dans l'état de la technique. L'avantage de ces dispositifs est qu'il n'est pas nécessaire de stocker l'ensemble des doses à l'intérieur de l'appareil, de sorte que celui-ci peut être de dimension réduite. Par contre, l'utilisation est plus complexe, puisque l'utilisateur est obligé de charger une capsule dans l'inhalateur avant chaque utilisation. Un autre type d'inhalateur consiste à disposer les doses de poudre dans des réservoirs individuels prédosés, puis d'ouvrir un de ces réservoirs à chaque actionnement de l'inhalateur. Cette mise en oeuvre assure une meilleure étanchéité de la poudre, puisque chaque dose n'est ouverte qu'au moment de son expulsion. Pour réaliser ces réservoirs individuels, diverses variantes ont déjà été proposées, telle qu'une bande de blisters allongée ou des blisters disposés sur un disque circulaire rotatif. Tous les types d'inhalateurs décrits ci-dessus et existants présentent des avantages et des inconvénients liés à leur structure et à leur fonctionnement. Ainsi, avec certains inhalateurs, se pose le problème de la précision et de la reproductibilité du dosage à chaque actionnement. De même, l'efficacité de la distribution, c'est-à-dire la partie de la dose qui pénètre effectivement dans les poumons de l'utilisateur pour avoir un effet thérapeutique bénéfique, est également un problème qui se présente avec un certain nombre d'inhalateurs. Une solution pour résoudre ce problème spécifique a été de synchroniser l'expulsion de la dose avec l'inhalation du patient. A nouveau, ceci pouvait générer des inconvénients, à savoir que généralement dans ce type de dispositif, la dose est d'abord chargée dans un conduit d'expulsion avant l'inhalation, puis l'expulsion est synchronisée avec l'inhalation. Ceci signifie que si l'utilisateur laisse tomber, secoue ou manipule de manière non souhaitée ou inadaptée l'inhalateur entre le moment où il a chargé la dose (soit à partir d'un réservoir multidoses, soit à partir d'un réservoir individuel) et au moment où il inhale, il risque de perdre toute ou partie de cette dose, celle-ci pouvant se répartir à l'intérieur de l'appareil. Dans ce cas il peut se présenter un gros risque de surdosage lors de la prochaine utilisation du dispositif. L'utilisateur qui se rendra compte que sa dose n'est pas complète chargera une nouvelle dose dans l'appareil, et lors de l'inhalation de cette nouvelle dose, une partie de la dose précédente perdue dans l'appareil pourrait être alors expulsée en même temps que la nouvelle dose, provoquant un surdosage. Selon les traitements envisagés, ce surdosage peut être très néfaste et c'est une exigence de plus en plus forte des autorités de tous les pays de limiter au maximum ce risque de surdosage. Un autre problème qui peut se poser concerne l'assemblage de certaines pièces, notamment mobiles, qui doivent supporter des contraintes importantes en fonctionnement, et pour lesquels l'assemblage doit être particulièrement fiable pour éviter tout risque de dysfonctionnement. Avec la petite taille de certaines pièces et des possibles déformations issues de moulage et/ou du conditionnement de composants plastiques, il peut être compliqué de garantir une telle fiabilité d'assemblage. Des assemblages étanches, notamment dans des inhalateurs utilisant un système de déclenchement par l'inhalation, sont souhaitables, notamment pour éviter toute perte de charge lors de l'inhalation, mais aussi pour assurer la répétabilité du seuil de déclenchement. Ceci améliore le confort d'utilisation des personnes malades et permet de ne pas perturber les habitudes des patients causées par d'éventuelles variations d'un inhalateur à l'autre. Les documents US-6 179 164, FR-2 936 425 et WO 02/085281 décrivent des dispositifs de l'art antérieur.

La présente invention a pour but de fournir un dispositif de distribution de produit fluide, en particulier un inhalateur de poudre sèche qui ne reproduit pas les inconvénients susmentionnés.

En particulier, la présente invention a pour but de fournir un tel dispositif qui soit simple et peu coûteux à fabriquer et à assembler, fiable à l'assemblage et en utilisation, garantissant une précision et une reproductibilité du dosage à chaque actionnement, fournissant un rendement optimal quant à l'efficacité du traitement, en permettant de distribuer une part importante de la dose au niveau des zones à traiter, en particulier les poumons, évitant de manière sûre et efficace les risques des surdosages, de dimensions aussi petites que possibles, tout en garantissant une étanchéité et une intégrité absolue de toutes les doses jusqu'à leur expulsion.

L'invention est définie par les revendications ci-jointes. La présente invention a donc pour objet un dispositif de distribution de produit fluide, comportant un corps principal, ledit dispositif comportant au moins un réservoir individuel contenant une dose unique de produit fluide, tel que de la poudre, des moyens d'ouverture étant prévus pour ouvrir un réservoir individuel à chaque actionnement du dispositif, un corps supérieur étant monté fixement sur ledit corps principal, ledit corps supérieur recevant un embout buccal définissant un orifice de distribution, ledit dispositif comportant un système de déclenchement par l'inhalation qui comporte une chambre d'air déformable coopérant avec ledit embout d'inhalation et un élément déclencheur coopérant avec ladite chambre d'air, de sorte que lors d'une inhalation à travers ledit embout d'inhalation, ladite chambre d'air est déformée et ledit élément déclencheur actionne lesdits moyens d'ouverture, de sorte que lors d'une inhalation à travers l'embout d'inhalation, un réservoir est ouvert par lesdits moyens d'ouverture, ledit embout buccal étant fixé audit corps supérieur, ledit embout buccal comportant une lèvre périphérique déformable coopérant, après assemblage, de manière étanche avec un bord périphérique chanfreiné dudit corps supérieur.

Avantageusement, ledit bord périphérique chanfreiné comporte une partie de bord supérieur et une partie de bord inférieur.

Avantageusement, les inclinaisons desdites parties de bord supérieur et inférieur sont différentes.

Avantageusement, ledit embout buccal comporte des fenêtres d'encliquetage adaptées à s'encliqueter sur des projections d'encliquetage dudit corps supérieur.

Avantageusement, chaque fenêtre d'encliquetage comporte une languette déformable qui est déformée par une projection d'encliquetage correspondante lors de l'assemblage.

Avantageusement, ledit corps supérieur comporte des plots de positionnement adaptés à coopérer, lors de l'assemblage, avec des logements de positionnements de l'embout buccal.

Avantageusement, le dispositif comporte des moyens de support mobiles adaptés à déplacer un réservoir individuel contre lesdits moyens d'ouverture à chaque actionnement, lesdits moyens de support mobiles étant déplaçables entre une position de non-distribution et une position de distribution, lesdits moyens de support mobiles étant sollicités vers leur position de distribution par des moyens élastiques, tel qu'un ressort ou une lame élastique, et étant retenus en position de non-distribution par des moyens de blocage qui sont libérés par l'inhalation de l'utilisateur.

Avantageusement, lesdits moyens d'ouverture comportent un élément de perçage fixe par rapport audit corps principal, adapté à découper une paroi de fermeture du réservoir de telle sorte que la ou les partie(s) découpée(s) n'obstrue(nt) pas la ou les ouverture(s) formée(s).

Avantageusement, le dispositif comporte au moins un élément de capot monté pivotant sur ledit corps principal entre une position fermée et une position ouverte.

Ces caractéristiques et avantages et d'autres de la présente invention apparaîtront plus clairement au cours de la description détaillée suivante, faite en référence aux dessins joints, donnés à titre d'exemples non limitatifs, sur lesquels
- la figure 1 est une vue schématique en section transversale d'un dispositif de distribution selon un mode de réalisation avantageux de l'invention, en position de repos,
- la figure 2 est une vue schématique de détail en section transversale d'une partie du dispositif de la figure 1,
- la figure 3 est une vue schématique en perspective du corps supérieur,
- les figures 4 et 5 sont des vues schématiques en perspective de l'embout buccal, respectivement de dessus et de dessous, et
- les figures 6 à 9 sont des vues schématiques partielles en section transversale illustrant la phase d'assemblage de l'embout buccal sur le corps supérieur.

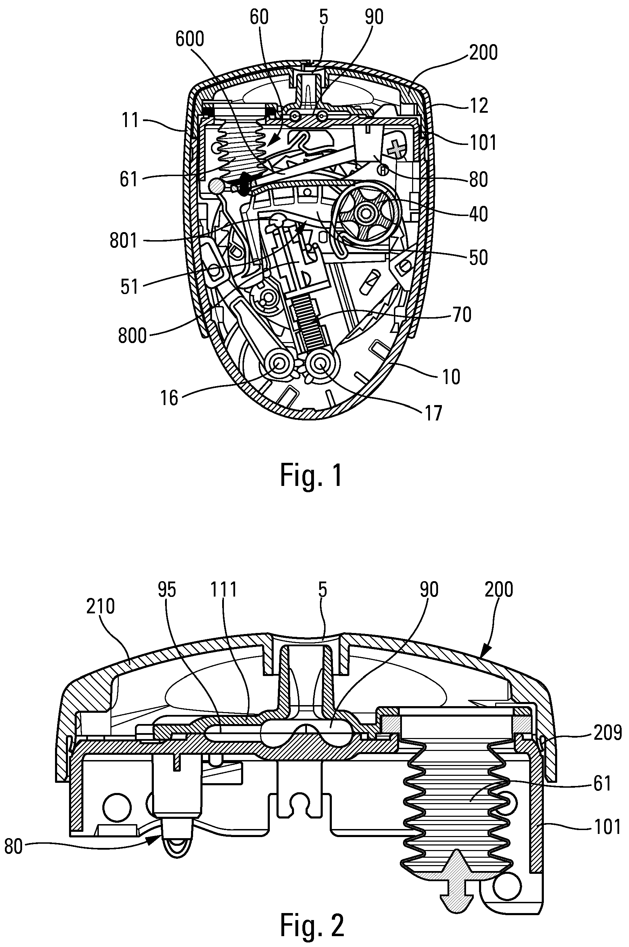

Sur les figures 1 et 2, il est représenté un exemple avantageux d'un inhalateur de poudre sèche. Cet inhalateur comporte un corps principal 10 sur lequel peuvent être montées coulissantes deux parties 11, 12 formant capot, adaptées à être ouvertes pour ouvrir et charger le dispositif. Le corps principal 10 peut être de forme environ arrondie comme représenté sur les figures, mais il pourrait avoir toute autre forme appropriée. Un corps supérieur 101 est assemblé au corps principal 10, et un embout buccal 200 est assemblé sur ledit corps supérieur 101. Cet embout buccal 200 définit un orifice de distribution 5 à travers lequel l'utilisateur va inhaler lors de l'actionnement du dispositif. Cet orifice de distribution 5 est typiquement disposé environ au centre de l'embout buccal 200. Les capots 11, 12 peuvent s'ouvrir par pivotement autour d'un axe de rotation commun, ou autour de deux axes parallèles en étant engrené l'un avec l'autre. Tout autre moyen d'ouverture du dispositif est envisageable. En variante, le dispositif pourrait ne comporter qu'un seul capot au lieu de deux.

A l'intérieur du corps principal 10, il est prévu une bande de réservoirs individuels (non représentée dans des buts de clarté), également appelés blisters, réalisée sous forme d'une bande allongée sur laquelle les blisters sont disposés les uns derrière les autres, de manière connue. Cette bande de blisters est avantageusement constituée d'une couche ou paroi de base formant les cavités recevant les doses de poudre, et d'une couche ou paroi de fermeture qui obture de manière étanche chacun desdits blisters. Avant la première utilisation, la bande de blisters peut être enroulée à l'intérieur du corps principal 10, de préférence dans une partie de stockage, et des premiers moyens de déplacement de bande 40, notamment rotatifs, sont prévus pour progressivement dérouler et faire avancer cette bande de blisters.

Des seconds moyens de déplacement 50, notamment pivotants sur le corps principal 10, sont prévus pour amener un blister respectif dans une position de distribution à chaque actionnement du dispositif. Ces seconds moyens de déplacement sont avantageusement pivotants entre une position de non-distribution et une position de distribution, dans laquelle un blister coopère avec lesdits moyens d'ouverture.

La partie de bande comportant les blisters vides est avantageusement adaptée à s'enrouler dans un autre endroit dudit corps 10, de préférence une partie de réception, comme cela sera décrit plus en détail ci-après.

L'inhalateur comporte des moyens d'ouverture de blister 80 comportant de préférence une aiguille de perçage et/ou de coupage de la couche de fermeture des blisters. De préférence, les moyens d'ouverture comportent un élément de perçage 80, fixe par rapport aux corps principal 10 et supérieur 101, et contre lequel un blister respectif est déplacé à chaque actionnement par les seconds moyens de déplacement. Le blister est alors percé par ledit élément de perçage, qui pénètre dans ledit blister pour expulser la poudre au moyen du flux d'inhalation de l'utilisateur. Avantageusement, l'élément de perçage est adapté à découper une paroi de fermeture du réservoir de telle sorte que la ou les partie(s) découpée(s) n'obstrue(nt) pas la ou les ouverture(s) formée(s). Les documents WO 2006/079750 et WO 2009/007640 décrivent de tels moyens d'ouverture de blister, et sont donc intégrés dans la présente description à titre de références.

Les premiers moyens de déplacement 40 sont adaptés à faire avancer la bande de blisters après chaque inhalation de l'utilisateur. Les seconds moyens de déplacement 50 sont adaptés à déplacer le blister à vider contre lesdits moyens d'ouverture lors de l'actionnement, avant chaque inhalation. Ces seconds moyens de déplacement peuvent être sollicités par un élément élastique 70, tel qu'un ressort ou tout autre élément élastique équivalent, ledit élément élastique pouvant être pré-chargé lors de l'ouverture du dispositif.

De préférence, les premiers moyens de déplacement 40 sont formés par une roue d'indexage qui reçoit et guide la bande de blisters. La suite de la description sera donc faite en référence à une telle roue d'indexage 40. Une rotation de cette roue d'indexage 40 fait avancer la bande de blisters. Avant chaque inhalation, un blister plein est toujours en position face aux moyens d'ouverture 80. Les seconds moyens de déplacement 50 peuvent comporter un organe pivotant autour d'un axe de rotation, ladite roue d'indexage 40 étant avantageusement montée rotative sur ledit organe pivotant.

Un cycle d'actionnement du dispositif peut être le suivant. Lors de l'ouverture du dispositif, les deux parties latérales 11, 12 formant capot sont écartées l'une de l'autre en pivotant sur le corps pour ouvrir le dispositif, et ainsi charger le dispositif. Dans cette position la roue d'indexage 40 ne peut pas se déplacer vers l'élément de perçage 80 car les seconds moyens de déplacement 50 sont retenus par des moyens de blocage appropriés (non représentés dans des buts de clarté). Les documents WO 2009/077700 et WO 2009/136098 décrivent de tels moyens de blocage, et sont donc intégrés dans la présente description à titre de références. Lors de l'inhalation par l'utilisateur à travers l'embout buccal, ces moyens de blocage sont débloqués, ce qui provoque alors le déplacement de ladite roue d'indexage 40 en direction de l'aiguille, et donc l'ouverture d'un blister.

Comme expliqué ci-dessus, il est souhaitable que l'actionnement des moyens d'ouverture soit réalisé par l'inhalation de l'utilisateur. Pour réaliser ce déclenchement par inhalation des moyens d'ouverture, on prévoit un système de déclenchement par l'inhalation 60 qui comporte avantageusement une chambre d'air 61 déformable sous l'effet de l'inhalation, cette chambre d'air étant adaptée à libérer les moyens de blocage. La chambre d'air 61 peut avantageusement être réalisée en forme de soufflet. L'inhalation de l'utilisateur provoque la déformation de ladite chambre d'air déformable, libérant ainsi lesdits moyens de blocage et permettant donc le déplacement des seconds moyens de déplacement, et donc d'un blister respectif, vers sa position d'ouverture. Le blister n'est donc ouvert qu'au moment de l'inhalation, de sorte qu'il est simultanément vidé. Il n'y a donc aucun risque de perte de dose entre l'ouverture du blister et son vidage.

L'inhalateur comporte en outre une chambre de dispersion 90 qui est destinée à recevoir la dose de poudre après ouverture d'un blister respectif. Avantageusement, cette chambre de dispersion est pourvue d'au moins une bille, de préférence plusieurs, qui se déplace(nt) à l'intérieur de ladite chambre 90 pendant l'inhalation, notamment pour améliorer la distribution du mélange air et poudre après ouverture d'un blister, afin d'augmenter l'efficacité du dispositif.

Il peut être avantageux que les moyens d'ouverture, en particulier l'élément de perçage, soient directement reliés à ladite chambre de dispersion, par exemple via un canal 95 menant à ladite chambre 90.

Après l'inhalation, lorsque l'utilisateur referme le dispositif, tous les composants reviennent vers leurs positions de repos initiales. Le dispositif est alors prêt pour un nouveau cycle d'utilisation.

Selon un aspect avantageux de l'inhalateur, les blisters sont formés sur une bande allongée souple, qui, au début, est principalement stockée sous forme de bobine dans un logement de stockage à l'intérieur du corps principal 10 du dispositif. Avantageusement, cette bande de blisters enroulée est maintenue par des parois internes dudit logement de stockage sans que son extrémité arrière (dans le sens d'avancement de la bande de blisters) ne soit fixée par rapport audit corps principal 10, ce qui permet un assemblage plus aisé de cette bobine de bande de blisters à l'intérieur du dispositif. La bande de blisters est déplacée au moyen de la roue d'indexage 40 qui présente avantageusement au moins un, de préférence plusieurs évidements, dont la forme correspond à celle des blisters. Ainsi, lorsque cette roue d'indexage 40 tourne, elle fait avancer la bande de blisters. Bien entendu en variante ou de manière additionnelle, on pourrait utiliser d'autres moyens d'avancée de bande de blisters, par exemple en prévoyant un profil sur les bords longitudinaux latéraux de la bande de blisters, ledit profil étant adapté à coopérer avec des moyens d'entraînement appropriés. De même, des trous ménagés le long des bords latéraux de la bande de blisters pourraient également être utilisés pour faire avancer la bande de blisters au moyen de roues dentées coopérant avec lesdits trous.

Après ouverture d'un ou plusieurs blister(s), la partie de bande de blisters avec les blisters vidés doit pouvoir être stockée de manière aisée et non encombrante dans le dispositif, en évitant tout risque de blocage. Avantageusement, cette bande de blisters usée s'enroule automatiquement sur elle-même pour à nouveau former une bobine.

Selon encore un autre aspect de l'inhalateur, un dispositif de comptage ou d'indication de doses (non représenté dans des buts de clarté) peut également être prévu. Ce dispositif peut comporter des chiffres ou symboles inscrits directement sur la bande de blister et visibles à travers une fenêtre appropriée dans le corps principal 10 du dispositif. En variante, on pourrait envisager d'utiliser un compteur avec un ou plusieurs disque(s) ou anneau(x) rotatif(s) comportant des numéros ou symboles. Les documents WO 2008/012458 et WO 2011/154659 décrivent de tels compteurs, et sont donc intégrés dans la présente description à titre de références. Un but de l'invention est d'éviter de compter des doses qui ne sont pas distribuées, par exemple en cas d'erreur de manipulation, ou d'une manipulation incomplète du dispositif. Il est donc souhaitable que le compteur ou indicateur ne soir actionné qu'une fois que l'utilisateur a inhalé, puisque c'est cette inhalation qui conditionne l'ouverture et la distribution de la dose de chaque blister. Avantageusement, l'actionnement du compteur se fait donc après inhalation, lorsque l'utilisateur referme le dispositif.

L'élément de capot mobile 12 est solidaire d'un organe d'armement 800 qui peut coulisser dans un logement approprié. Cet organe d'armement 800 est avantageusement pivotant par rapport audit corps 10 ensemble avec l'élément de capot 12. Cet organe d'armement 800 peut se déplacer contre le ressort 70, avantageusement un ressort à boudins, disposé dans ledit logement. L'organe d'armement 800 est donc connecté d'un côté audit ressort 70 et de l'autre côté, il coopère avec les seconds moyens de déplacement, en particulier avec un organe 50 pivotant sur le corps 10, et sur lequel est fixée de manière rotative la roue d'indexage 40.

Lorsque l'élément de capot mobile 12 est ouvert, l'organe d'armement 800 est déplacé dans son logement en comprimant le ressort 70. L'organe pivotant 50 des seconds moyens de déplacement est lui empêché de se déplacer par les moyens de blocage susmentionnés, qui ne seront libérés qu'au moment de l'inhalation. Ainsi, en l'absence d'inhalation dans la position ouverte, la fermeture des éléments de capot 11, 12 provoquerait simplement le retour à la position de repos pour l'organe d'armement 800, et la décompression du ressort 70.

Ainsi, lorsque l'utilisateur ouvre l'inhalateur il charge le système. S'il n'inhale pas et qu'il referme l'inhalateur, celui-ci reprend simplement sa position de départ sans déplacer la bande de blisters ni les moyens de blocage. Il n'y a donc aucun risque qu'un blister (et donc une dose de produit actif) soit perdu par un actionnement malencontreux ou incomplet dans lequel l'utilisateur n'exercerait pas d'inhalation entre l'ouverture et la fermeture. L'ouverture du blister, son vidage, la distribution de la poudre dans les poumons de l'utilisateur, le déplacement de la bande de blisters pour amener un nouveau blister plein en face des moyens d'ouverture ainsi que le comptage de la dose ne sont donc possibles que si l'utilisateur inhale.

Les moyens de blocage, qui bloquent les seconds moyens de déplacement et notamment l'organe pivotant qui coopère avec l'organe d'armement, sont connectés à la chambre d'air déformable 61 sensible à l'inhalation de l'utilisateur via un élément déclencheur 600, de sorte que lors de l'inhalation de l'utilisateur, ladite chambre d'air déformable se déforme, faisant pivoter ledit élément déclencheur 600 et libérant lesdits moyens de blocage. Ceci permet le déplacement desdits seconds moyens de déplacement vers leur position de distribution sous l'effet de la force exercée par le ressort comprimé 70 sur l'organe d'armement 800 qui pousse sur l'organe pivotant 50. Ce déplacement provoque l'ouverture d'un blister plein et la distribution d'une dose.

Une surface de came 51 est formée sur lesdits moyens de support mobiles 50, sur laquelle glisse l'organe d'armement 800. L'organe d'armement 800 est donc adaptée à comprimer le ressort 70 lorsque l'élément de capot 12 est ouvert, et à décomprimer ledit ressort 70 lorsque ledit élément de capot 12 est refermé.

Avantageusement, l'organe d'armement 800 comporte, dans sa partie en contact avec la surface de came 51, une partie arrondie 801 pour favoriser le glissement de l'organe d'armement 800 sur ladite surface de came 51.

Les moyens de support mobiles sont dans ce mode de réalisation réalisés sous la forme d'un organe 50 monté pivotant sur le corps 10 autour d'un axe de pivotement. La surface de came précitée 51 étant formée sur ledit organe pivotant 50, lorsque le ressort 70 est chargé lors de l'ouverture de l'élément de capot mobile 12, ledit organe pivotant 50 est sollicité vers sa position de distribution par ledit organe d'armement 800 et le ressort 70 comprimé.

La surface de came 51 peut comporter au moins deux parties de pente différentes, avantageusement séparées par un sommet. En partant à nouveau de la position fermée de l'élément de capot mobile, la première partie de pente sur laquelle coulisse l'organe d'armement 800 permet la compression du ressort 70 comme décrit précédemment. Lorsque le ressort est chargé, c'est-à-dire comprimé, la surface de came 51 peut prévoir une seconde partie de pente différente avec laquelle l'organe d'armement 800 va coopérer uniquement en position d'ouverture du dispositif. De préférence, l'organe d'armement 800 exerce une force sensiblement perpendiculaire sur cette seconde partie de surface de came. De cette manière, la position chargée est stable. En variante, la seconde partie de pente forme un cran de butée dans laquelle l'organe d'armement 800 vient se positionner en position ouverte.

Après l'inhalation, c'est-à-dire en position de distribution, les moyens de blocage ont été libérés et les moyens de support mobiles 50 ont été déplacés vers le haut par le ressort comprimé 70.

Avantageusement, les deux éléments de capot mobiles 11, 12 sont engrenés l'un dans l'autre via des engrenages appropriés pour garantir une ouverture et une fermeture symétrique desdits deux éléments de capot mobiles. Cet engrenage peut se faire à proximité de leur axe de pivotement 16, 17.

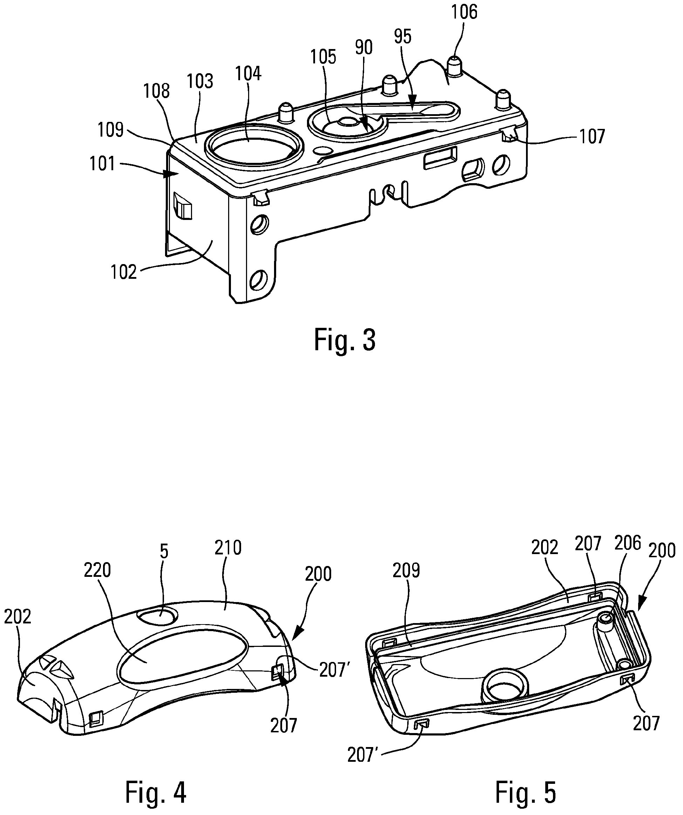

La figure 3 représente plus particulièrement le corps supérieur 101. Celui-ci comprend une paroi latérale périphérique 102 qui est fixée au corps principal 10, et une paroi supérieure 103 sensiblement plane. La paroi latérale 102 est de forme environ rectangulaire dans l'exemple de la figure 3, mais une forme différente est aussi possible. La paroi supérieure 103 comprend une ouverture 104 destinée à recevoir la chambre d'air déformable 61 du système de déclenchement par l'inhalation 60. La paroi supérieure 103 comprend aussi un évidement 105 définissant le fond de la chambre de dispersion 90 et du canal 95 associé. Un partie de couvercle 111 est destinée à recouvrir ledit évidement 105, comme visible sur la figure 2. La paroi supérieure 103 comporte aussi des plots de positionnement 106 destinés à coopérer avec des logements de positionnements correspondants prévus dans le fond de l'embout buccal, comme cela sera décrit ci-après. A la jonction entre la paroi latérale 102 et la paroi supérieure 103, il est prévu un bord périphérique. Ce bord périphérique est de préférence chanfreiné, et comporte avantageusement une partie de bord supérieur 108, au contact de la paroi supérieure 103, et une partie de bord inférieur 109, au contact de la paroi latérale 102. Avantageusement, les inclinaisons de ces parties de bord supérieur et inférieur peuvent être différentes. Le corps supérieur 101 comporte en outre des projections d'encliquetage 107, adaptés à coopérer avec des fenêtres d'encliquetage correspondantes de l'embout buccal, pour assurer la fixation de l'embout buccal sur le corps supérieur, comme cela sera décrit ci-après. Ces projections d'encliquetage 107 sont avantageusement disposées sur la paroi latérale 102, au niveau de la partie de bord inférieur 109.

Les figures 4 et 5 représentent plus particulièrement l'embout buccal 200. De manière générale, l'embout buccal 200 est de préférence symétrique par rapport audit orifice de distribution 5. Il comporte une paroi latérale périphérique 202, dont la forme est adaptée à celle de la paroi latérale périphérique 102 du corps supérieur 101, et une paroi supérieure 210. Cette paroi supérieure 210 s'étend longitudinalement des deux côtés de l'orifice de distribution 5. On constate que cette paroi supérieure 210 est également légèrement bombée ou convexe dans la direction longitudinale, octroyant ainsi à l'embout buccal 200 une forme arrondie aplatie. De part et d'autre de l'orifice de distribution 5, dans la direction transversale ou latérale, l'embout buccal comporte deux surfaces d'appui latérales 220 respectivement prévues latéralement de chaque côté dudit orifice de distribution 5. Chacune de ces surfaces d'appui latérales 220 est destinée à recevoir une lèvre de l'utilisateur lors de l'inhalation. Ces surfaces d'appui latérales 220 sont légèrement concaves, notamment dans la direction transversale, pour former de légers creux adaptés à bien positionner les lèvres de l'utilisateur lorsque celui-ci les place sur l'embout buccal. Comme visible notamment sur la figure 4, les surfaces d'appui latérales 220 prolongent latéralement ladite paroi supérieure 210, et sont de préférence séparées l'une de l'autre par cette paroi supérieure 210, ainsi que par l'orifice de distribution 5. De cette manière, lorsque l'utilisateur place sa bouche sur l'embout buccal 200, l'écartement entre les deux surfaces d'appui latérales 220 oblige l'utilisateur à ouvrir suffisamment la bouche pour éviter que ses dents ne viennent obturer même partiellement l'orifice de distribution 5 lors de l'inhalation. Par ailleurs, la forme légèrement concave des surfaces d'appui latérales 220 garantit une bonne ergonomie et assure une bonne étanchéité de la bouche de l'utilisateur sur l'embout buccal 200 au moment de l'inhalation. Ceci évite notamment une perte de charge lors de l'inhalation et garantit que la plus grande partie possible de l'écoulement d'inhalation crée par l'utilisateur sera utilisée pour actionner l'inhalateur et expulser la poudre contenue dans l'inhalateur. L'écartement et la profondeur des surfaces d'appui latérales 220 garantit également que l'orifice de distribution se positionne à l'intérieur de la bouche, au-delà des dents, sans pour autant que cet orifice de distribution 5 soit formé sur une partie saillante de l'embout buccal 200.

Avantageusement, les surfaces d'appui latérales 220 s'étendent longitudinalement sur une partie centrale dudit embout buccal 200, de préférence sur la plus grande partie de celui-ci, comme cela est notamment visible sur la figure 4. Avantageusement, ces deux surfaces d'appui latérales 220 sont parfaitement symétriques l'une par rapport à l'autre par rapport à l'orifice de distribution 5.

En se référant à nouveau à la figure 1, on constate que les éléments de capot 11, 12 mobiles autour du corps principal 10 de l'inhalateur et de l'embout buccal 200, comportent une surface supérieure de forme bombée sensiblement adaptée à celle de l'embout buccal, notamment à sa paroi supérieure 210. De cette manière, lors du déplacement desdits éléments de capot entre la position d'ouverture et de fermeture, aucun espace n'est crée par la présence de l'embout buccal 200, de sorte que l'utilisateur ne risque pas de se coincer les doigts lors de la manipulation de ces éléments de capot. De manière générale, l'embout buccal ne comporte aucune partie saillante qui risquerait de créer un interstice pouvant recevoir des doigts de l'utilisateur lors de l'actionnement du dispositif.

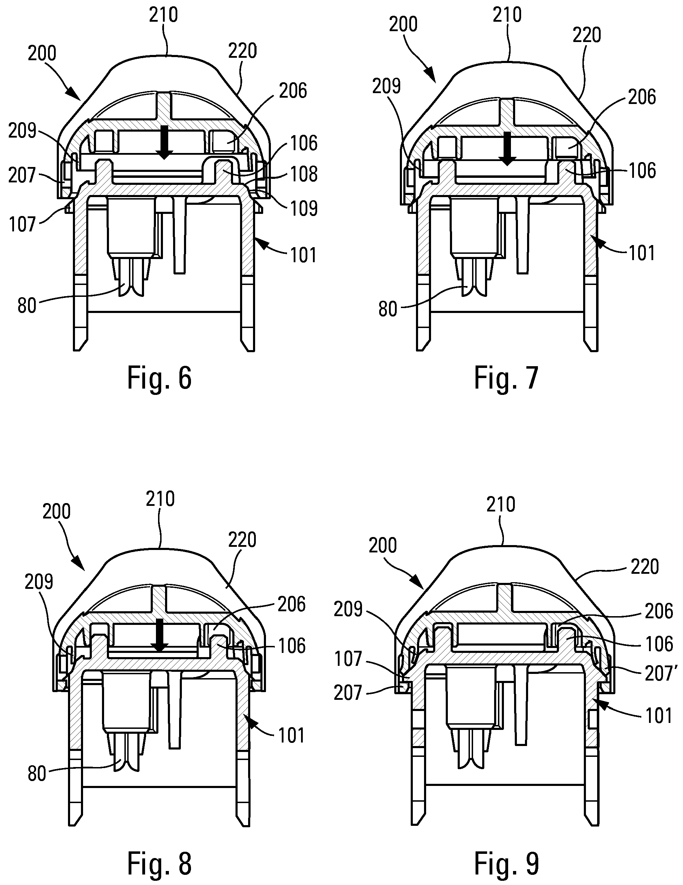

La paroi latérale 202 de l'embout buccal 200 comporte les fenêtres d'encliquetage 207 susmentionnées qui vont s'encliqueter sur les projections d'encliquetage 107 du corps supérieur lors de l'assemblage de l'embout buccal sur ledit corps supérieur. Avantageusement, il y a quatre fenêtres 207 pour quatre projections 107. Avantageusement, chaque fenêtre d'encliquetage 207 peut comporter une languette déformable 207' qui sera déformée, notamment radialement vers l'extérieur, par une projection respective, comme visible sur la figure 9.

La paroi supérieure 210 comporte sur sa surface interne les logements de positionnement 206 adaptés à coopérer avec les plots de positionnement 106 lors de l'assemblage de l'embout buccal. Ceci garantit un positionnement précis de l'embout buccal 200 par rapport au corps supérieur 101 au moment de l'assemblage.

Selon l'invention, l'embout buccal 200 comporte une lèvre périphérique interne déformable 209 adaptée à coopérer avec le bord chanfreiné 108, 109 du corps supérieur 101. Cette lèvre 209 se déforme lors de l'assemblage pour améliorer l'étanchéité de cet assemblage.

Avantageusement, lors de l'assemblage, la lèvre 209 coopère d'abord avec la partie de bord supérieur 108, radialement plus à l'intérieur et moins inclinée (par rapport à l'horizontale dans la position des figures 2 et 6 à 9). Ceci garantit un bon guidage périphérique de la lèvre 209 sur la partie de bord inférieur 109, où sera réalisé le contact étanche après assemblage.

Les figures 6 à 9 illustrent la phase d'assemblage. Sur la figure 6, l'embout buccal 200 est positionné au-dessus du corps supérieur 101, et déplacé vers le bas dans le sens de la flèche. Sur la figure 7, les plots de positionnement 106 coopèrent avec les logements de positionnement 206 pour aligner l'embout buccal 200. En continuant l'assemblage, les plots 106 vont donc progressivement pénétrer dans les logements respectifs. Parallèlement, le bord inférieur des fenêtres d'encliquetage 207 vient au contact des projections d'encliquetage 207, et la lèvre 209 vient au contact de la partie de bord supérieur 108, comme visible sur la figure 8.

Après assemblage, représenté sur la figure 9, les plots 106 sont dans les logements 206, les fenêtres 207 sont encliquetées sur les projections 107, avec les languettes déformables 207' déformées, et la lèvre 209 est en contact périphérique étanche avec la partie de bord inférieur 109.

Avantageusement, les languettes déformables 107' garantissent la stabilité et la répétabilité du positionnement sous contrainte de l'embout buccal 200 sur le corps supérieur 101. Ainsi, la lèvre 209 a pour vocation unique de garantir l'étanchéité. Ceci a pour avantage d'offrir des leviers d'optimisation fonctionnels indépendants, comme par exemple l'optimisation de la qualité du positionnement sans risque de dégrader la fonction d'étanchéité.

Les formes, dimensions et positions des fenêtres 207, projections 107 et languettes déformables 207' peuvent être ajustées pour optimiser l'assemblage. La lèvre 209 peut avoir un profil approprié pour optimiser sa coopération d'étanchéité avec le bord périphérique du corps supérieur.

La présente invention permet donc de fournir un inhalateur de poudre sèche qui procure notamment les fonctions suivantes :
▪ une pluralité de doses individuelles de poudre stockées dans des blisters individuels étanches, par exemple 30 ou 60 doses stockées sur une bande enroulée en bobine ;
▪ la poudre libérée au moyen d'un perçage actionné par l'inhalation de l'utilisateur, ce perçage du blister étant réalisé au moyen d'un système de détection d'inhalation couplé à un système de libération pré-chargé ;
▪ des moyens d'entraînement de forme appropriée en prises avec les blisters pour réaliser le déplacement de la bande de blisters après chaque inhalation, et amener un nouveau blister plein dans une position dans laquelle il est destiné à être ouvert par les moyens d'ouverture appropriés ;
▪ des moyens pour éviter une perte de dose en cas d'ouverture de l'inhalateur mais en l'absence d'inhalation ;
▪ un indicateur de doses adapté à compter les doses seulement en cas d'inhalation.

D'autres fonctions sont également fournies par le dispositif de l'invention tel que cela a été décrit précédemment.

Il est à noter que les différentes fonctions, même si elles ont été représentées comme prévues simultanément sur l'inhalateur, pourraient être mises en oeuvre séparément les unes des autres. En particulier, le mécanisme de déclenchement par inhalation pourrait être utilisé indépendamment du type de moyens d'ouverture de réservoir, indépendamment de l'utilisation d'un indicateur de doses, indépendamment de la manière dont les blisters individuels sont arrangés les uns par rapport aux autres, etc. Les moyens d'armement et le système de déclenchement par l'inhalation pourraient être réalisés différemment. Il en est de même des autres parties constitutives du dispositif.

Diverses modifications sont également possibles pour un homme du métier sans sortir du cadre de la présente invention tel que défini par les revendications annexées. En particulier, les diverses caractéristiques et fonctionnalités du dispositif décrites en référence aux dessins peuvent être combinées entre elles d'une quelconque façon appropriée.

## Revendications

1. Dispositif de distribution de produit fluide, comportant un corps principal (10), ledit dispositif comportant au moins un réservoir individuel contenant une dose unique de produit fluide, tel que de la poudre, des moyens d'ouverture (80) étant prévus pour ouvrir un réservoir individuel à chaque actionnement du dispositif, un corps supérieur (101) étant monté fixement sur ledit corps principal (10), ledit corps supérieur (101) recevant un embout buccal (200) définissant un orifice de distribution, ledit dispositif comportant un système de déclenchement par l'inhalation (60) qui comporte une chambre d'air déformable (61) coopérant avec ledit embout d'inhalation (200) et un élément déclencheur (600) coopérant avec ladite chambre d'air (61), de sorte que lors d'une inhalation à travers ledit embout d'inhalation (200), ladite chambre d'air (61) est déformée et ledit élément déclencheur (600) actionne lesdits moyens d'ouverture (80), de sorte que lors d'une inhalation à travers l'embout d'inhalation (200), un réservoir est ouvert par lesdits moyens d'ouverture, **caractérisé en ce que** ledit embout buccal (200) est fixé audit corps supérieur (101), ledit embout buccal comportant une lèvre périphérique déformable (209) coopérant, après assemblage, de manière étanche avec un bord périphérique chanfreiné (108, 109) dudit corps supérieur (101).

2. Dispositif selon la revendication 1, dans lequel ledit bord périphérique chanfreiné comporte une partie de bord supérieur (108) et une partie de bord inférieur (109).

3. Dispositif selon la revendication 2, dans lequel les inclinaisons desdites parties de bord supérieur et inférieur sont différentes.

4. Dispositif selon la revendication 3, dans lequel ladite partie de bord supérieure (108) est radialement plus à l'intérieur et moins inclinée par rapport à ladite partie de bord inférieur (109.

5. Dispositif selon la revendication 4, dans lequel lors de l'assemblage, ladite lèvre déformable (209) coopère d'abord avec ladite partie de bord supérieur (108) pour assurer le guidage de ladite lèvre déformable (209) sur ladite partie de bord inférieur (109), où sera réalisée l'étanchéité après assemblage.

6. Dispositif selon l'une quelconque des revendications précédentes, dans lequel ledit embout buccal (200) comporte des fenêtres d'encliquetage (207) adaptées à s'encliqueter sur des projections d'encliquetage (107) dudit corps supérieur (101).

7. Dispositif selon la revendication 6, dans lequel chaque fenêtre d'encliquetage (207) comporte une languette déformable (207') qui est déformée par une projection d'encliquetage (107) correspondante lors de l'assemblage.

8. Dispositif selon l'une quelconque des revendications précédentes, dans lequel ledit corps supérieur (101) comporte des plots de positionnement (106) adaptés à coopérer, lors de l'assemblage, avec des logements de positionnements (206) de l'embout buccal (200).

9. Dispositif selon l'une quelconque des revendications précédentes, comportant des moyens de support mobiles (50) adaptés à déplacer un réservoir individuel contre lesdits moyens d'ouverture (80) à chaque actionnement, lesdits moyens de support mobiles (50) étant déplaçables entre une position de non-distribution et une position de distribution, lesdits moyens de support mobiles (50) étant sollicités vers leur position de distribution par des moyens élastiques (70), tel qu'un ressort ou une lame élastique, et étant retenus en position de non-distribution par des moyens de blocage qui sont libérés par l'inhalation de l'utilisateur.

10. Dispositif selon l'une quelconque des revendications précédentes, dans lequel lesdits moyens d'ouverture (80) comportent un élément de perçage fixe par rapport audit corps principal (10), adapté à découper une paroi de fermeture du réservoir de telle sorte que la ou les partie(s) découpée(s) n'obstrue(nt) pas la ou les ouverture(s) formée(s).

11. Dispositif selon l'une quelconque des revendications précédentes, comportant au moins un élément de capot (11, 12) monté pivotant sur ledit corps principal (10) entre une position fermée et une position ouverte.

## Patentansprüche

1. Vorrichtung zur Ausgabe eines Fluidprodukts mit einem Hauptkörper (10), wobei die Vorrichtung mindestens einen einzelnen Behälter aufweist, der eine Einzeldosis des Fluidprodukts, wie Pulver, enthält, wobei Öffnungseinrichtungen (80) vorgesehen sind, um einen einzelnen Behälter bei jeder Betätigung der Vorrichtung zu öffnen, wobei ein oberer Körper (101) fest an dem Hauptkörper (10) angebracht ist, wobei der obere Körper (101) einen Mundansatz (200) aufnimmt, der eine Ausgabeöffnung definiert, wobei die Vorrichtung ein System zum Auslösen durch Inhalation (60) aufweist, das eine verformbare Luftkammer (61) für das Zusammenwirken mit dem Inhalationsansatz (200) und ein Auslöseelement (600) für das Zusammenwirken mit der Luftkammer (61) aufweist, so dass bei einer Inhalation durch den Inhalationsansatz (200) die Luftkammer (61) verformt wird und das Auslöseelement (600) die Öffnungseinrichtungen (80) betätigt, so dass bei einer Inhalation durch den Inhalationsansatz (200) ein Behälter durch die Öffnungseinrichtungen geöffnet wird, **dadurch gekennzeichnet, dass** der Mundansatz (200) an dem oberen Körper (101) befestigt ist, wobei der Mundansatz eine verformbare umlaufende Lippe (209) aufweist, die nach der Montage mit einem abgefasten umlaufenden Rand (108, 109) des oberen Körpers (101) abdichtend zusammenwirkt.

2. Vorrichtung nach Anspruch 1, wobei der abgefaste umlaufende Rand einen oberen Randteil (108) und einen unteren Randteil (109) aufweist.

3. Vorrichtung nach Anspruch 2, wobei die Neigungen des oberen und unteren Randteils verschieden sind.

4. Vorrichtung nach Anspruch 3, wobei der obere Randteil (108) relativ zu dem unteren Randteil (109) radial weiter innen und weniger geneigt ist.

5. Vorrichtung nach Anspruch 4, wobei bei der Montage die verformbare Lippe (209) mit dem oberen Randteil (108) zusammenwirkt, um die Führung der verformbaren Lippe (209) auf dem unteren Randteil (109) sicherzustellen, wo nach der Montage die Abdichtung realisiert wird.

6. Vorrichtung nach einem der vorhergehenden Ansprüche, wobei der Mundansatz (200) Einrastfenster (207) aufweist, die eingerichtet sind, um an Einrastüberständen (107) des oberen Körpers (101) einzurasten.

7. Vorrichtung nach Anspruch 6, wobei jedes Einrastfenster (207) eine verformbare Zunge (207') aufweist, die bei der Montage durch einen entsprechenden Einrastüberstand (107) verformt wird.

8. Vorrichtung nach einem der vorhergehenden Ansprüche, wobei der obere Körper (101) Positionierstifte (106) aufweist, die eingerichtet sind, um bei der Montage mit Positionieraufnahmen (206) des Mundansatzes (200) zusammenzuwirken.

9. Vorrichtung nach einem der vorhergehenden Ansprüche, die bewegliche Trageeinrichtungen (50) aufweist, die eingerichtet sind, um einen einzelnen Behälter bei jeder Betätigung gegen die Öffnungseinrichtungen (80) zu bewegen, wobei die beweglichen Trageeinrichtungen (50) bewegbar sind zwischen einer Nichtausgabeposition und einer Ausgabeposition, wobei die beweglichen Trageeinrichtungen (50) durch elastische Einrichtungen (70), wie eine Feder oder eine Federlasche, in ihre Ausgabeposition beaufschlagt werden und durch Blockiereinrichtungen, die bei der Inhalation des Benutzers gelöst werden, in Nichtausgabeposition zurückgehalten werden.

10. Vorrichtung nach einem der vorhergehenden Ansprüche, wobei die Öffnungseinrichtungen (80) ein Durchstoßelement aufweisen, das relativ zu dem Hauptkörper (10) festgelegt ist und eingerichtet ist, um eine Verschlusswand des Behälters so zu stanzen, dass der ausgestanzte Teil bzw. die ausgestanzten Teile die gebildete(n) Öffnung(en) nicht blockieren.

11. Vorrichtung nach einem der vorhergehenden Ansprüche, die mindestens ein Kappenelement (11, 12) aufweist, das auf dem Hauptkörper (10) zwischen einer geschlossenen Position und einer geöffneten Position drehbar gelagert ist.

## Claims

1. A fluid dispenser device including a main body (10), said device including at least one individual reservoir containing a single dose of fluid, such as powder, opening means (80) being provided for opening an individual reservoir each time the device is actuated, an upper body (101) being mounted in stationary manner on said main body (10), said upper body (101) receiving a mouthpiece (200) that defines a dispenser orifice, said device including an inhalation trigger system (60) that comprises a deformable air chamber (61) that co-operates with said inhalation piece (200), and a trigger element (600) that co-operates with said air chamber (61), such that during inhalation through said inhalation piece (200), said air chamber (61) is deformed and said trigger element (600) actuates said opening means (80), such that during inhalation through the inhalation piece (200), a reservoir is opened by said opening means, the fluid dispenser device being **characterized in that** said mouthpiece (200) is fastened to said upper body (101), said mouthpiece including a deformable peripheral lip (209) that, after assembly, co-operates in leaktight manner with a beveled peripheral edge (108, 109) of said upper body (101).

2. A device according to claim 1, wherein said beveled peripheral edge comprises a top edge portion (108) and a bottom edge portion (109).

3. A device according to claim 2, wherein the slopes of said top and bottom edge portions are different.

4. A device according to claim 3, wherein said top edge portion (108) is radially further in and has a smaller slope compared to said bottom edge portion (109).

5. A device according to claim 4, wherein, during assembly, said deformable lip (209) co-operates initially with said top edge portion (108), so as to guarantee that said deformable lip (209) is guided over said bottom edge portion (109) where sealing is achieved after assembly.

6. A device according to any preceding claim, wherein said mouthpiece (200) includes snap-fastener slots (207) that are adapted to snap-fasten on snap-fastener projections (107) of said upper body (101).

7. A device according to claim 6, wherein each snap-fastener slot (207) includes a deformable tab (207') that is deformed by a corresponding snap-fastener projection (107) during assembly.

8. A device according to any preceding claim, wherein said upper body (101) includes positioner lugs (106) that, during assembly, are adapted to co-operate with positioner housings (206) of the mouthpiece (200).

9. A device according to any preceding claim, including movable support means (50) that are adapted to move an individual reservoir against said opening means (80) on each actuation, said movable support means (50) being displaceable between a non-dispensing position and a dispensing position, said movable support means (50) being urged towards their dispensing position by resilient means (70), such as a spring or a spring blade, and being held in their non-dispensing position by blocking means that are released by the user inhaling.

10. A device according to any preceding claim, wherein said opening means (80) include a perforator element that is stationary relative to said main body (10) and that is adapted to cut a closure wall of the reservoir in such a manner that the cut portion(s) does/do not obstruct the opening(s) that is/are formed.

11. A device according to any preceding claim, including at least one cover element (11, 12) that is mounted to pivot on said main body (10) between a closed position and an open position.
